# EUROPEAN PATENT APPLICATION

(11) **EP 4 389 049 A1**
(43) Date of publication of application: **26.06.2024**
(21) Application number: 22307004.6
(22) Date of filing: 22.12.2022
(51) Int. Cl.: A61B 34/30, A61B 34/37, A61B 34/00, A61B 34/20

(54) **ROBOTIC TRAJECTORY AXIS ADJUSTMENT INTERFACE**

(71) Applicant: Ecential Robotics, 38610 Gieres (FR)
(72) Inventor: SIEFFERT, Jérôme, 38610 GIERES (FR); VIDAL, Clément, 38610 GIERES (FR)
(74) Representative: Regimbeau

(57) **Abstract**

A robotic surgical system including a robotic arm, the robotic surgical system comprising: a handgrip (160), a tool guide (140) and a controller. The handgrip is supported by the robotic arm, the handgrip having a first trajectory button (162) and a second trajectory button (164). The tool guide supported by the handgrip. A controller having a memory and a processor, the memory storing at least one planned trajectory associated with a surgical procedure. The first trajectory button, when engaged, causes the processor of the controller to move the tool support away from the patient along a planned trajectory. The second trajectory button, when engaged, causes the processor of the controller to move the tool support toward the patient along the planned trajectory keeping a longitudinal axis of the tool support substantially coaxially aligned with the planned trajectory.

## Description

### BACKGROUND

A computer-assisted surgical system may include a robotic arm, controller, and navigational system. Robotic or robot-assisted surgeries have many associated advantages, particularly in terms of precise placement of surgical tools and/or implants. For example, during robot-assisted spine surgery, a trajectory is pre-planned for a tool or series of tools attached to the robotic arm via a tool guide based on a surgical plan. During surgery, the robot arm moves the tool guide, and by association a tool placed in or attached to the tool guide, to and along the pre-planned trajectory placing the tool guide and the tool a pre-planned distance from a patient. During surgery, the surgeon may want to adjust the distance of the tool from the patient to improve visibility or access, for instance. However, it is important that the tool maintain orientation on the pre-planned trajectory.

Accordingly, there is a need for systems, devices, and methods that improve computer-assisted surgical systems, for instance, by facilitating movement of the tool placed in or attached to the tool guide while maintaining orientation of the tool on the pre-planned trajectory.

### SUMMARY

Systems, methods, and devices are described for robotic surgical systems. Some embodiments of the invention provide a surgical robot (and optionally a navigation system) that utilizes a positioning system that allows movement of a tool guide to be controlled along a pre-planned trajectory where a longitudinal axis of the tool guide, and by association a surgical instrument secured in the tool guide, is maintained coaxially aligned with the pre-planned trajectory throughout the movement. In some embodiments, the surgical robot can include a base, a robotic arm coupled to and configured for articulation relative to the base, as well as a handgrip and tool guide coupled to a distal end of the robot arm.

In some embodiments, the present disclosure describes a robotic surgical system including a robotic arm, the robotic surgical system comprising: a handgrip supported by the robotic arm, the handgrip having a first trajectory button and a second trajectory button; a tool guide supported by the handgrip, the tool guide comprising a tool support having a first end, a second end, an aperture extending through the tool support from the first end to the second end, and a longitudinal axis extending through a center of the aperture from the first end to the second end; and a controller in communication with the robotic arm and the handgrip, the controller having a non-transitory computer readable memory and a processor, the non-transitory computer readable memory storing at least one planned trajectory associated with a surgical procedure and processor executable instructions that, when executed, cause the processor to pass a first signal to the robotic arm causing the robotic arm to position the tool support at a predetermined distance from a patient with the longitudinal axis of the tool support substantially coaxially aligned with the at least one planned trajectory; wherein the first trajectory button, when engaged, is configured to pass a second signal to the processor of the controller causing the processor of the controller to move the tool support away from the patient along the at least one planned trajectory keeping the longitudinal axis of the tool support coaxially aligned with the at least one planned trajectory and the second trajectory button, when engaged, is configured to pass a third signal to the processor of the controller causing the processor of the controller to move the tool support toward the patient along the at least one planned trajectory keeping the longitudinal axis of the tool support coaxially aligned with the at least one planned trajectory.

In some embodiments, the robotic surgical system further comprises a tool positioned in the tool support.

In some embodiments, the non-transitory computer readable memory of the controller further stores a safety limit associated with the surgical procedure, the safety limit configured to restrict movement of the tool secured in the tool support such that a predetermined distance limit between the tool and anatomy of a patient is maintained during the surgical procedure.

In some embodiments, the handgrip further comprises a first admittance button and a second admittance button, the first admittance button and second admittance button, when engaged at substantially the same time, are configured to pass a fourth signal to the controller causing the controller to allow a user to manually move the robotic arm in various directions.

In some embodiments, the robotic surgical system further comprises a navigation system having a tracking unit and a navigation array attached to the robotic arm, the navigation system in communication with the controller, wherein the navigation system is configured to track a position and orientation of the tool guide using the navigation array and pass the position and orientation of the tool guide to the controller.

In some embodiments, the robotic surgical system further comprises a sterile drape disposed between the tool guide and the handgrip and extending to cover the handgrip during a surgical procedure.

In some embodiments, the first trajectory button and the second trajectory button, when engaged in a predetermined sequence, are configured to pass a fourth signal to the processor of the controller causing the processor of the controller to move the tool guide from a current position to a new position with the longitudinal axis of the tool guide coaxially aligned with a second planned trajectory.

In some embodiments, the first trajectory button and the second trajectory button are positioned opposite each other on the handgrip.

The first admittance button and second admittance button may be positioned opposite each other on the handgrip with the first admittance button offset from the first trajectory button.

In some embodiments, the handgrip further comprises a body supporting the first trajectory button, the second trajectory button, the first admittance button and the second admittance button, and the body of the handgrip comprises a ridge positioned to separate the first trajectory button and the second trajectory button from the first admittance button and second admittance button.

In some embodiments, the first trajectory button and the second trajectory button are provided having a first color and the first admittance button and second admittance button are provided having a second color different from the first color.

In some embodiments, the robotic surgical system further comprises a safety signal generator having circuitry configured to monitor a predetermined location and detect a presence or absence of a part of the surgeon in the predetermined area, and the processor is programmed to require the safety signal generator to be pushed or otherwise selected in a predetermined sequence with the second trajectory button to cause the processor of the controller to move the tool support toward the patient along the at least one planned trajectory.

In some embodiments, the robotic surgical system further comprises a safety signal generator having circuitry configured to monitor a predetermined location and detect a presence or absence of a part of the surgeon in the predetermined area, and the processor is programmed to require the safety signal generator to be pushed or otherwise selected in a predetermined sequence with the first trajectory button to cause the processor of the controller to move the tool support away from the patient along the at least one planned trajectory.

Another object of the present disclosure is a method of assembling a surgical robotic system as described above, comprising:
attaching a handgrip to at least one arm segment of a robotic arm, the handgrip having a body supporting a first trajectory button and a second trajectory button.

In some embodiments, one of the arm segments of the robotic arm forms a distal end of the robotic arm, and the step of attaching is defined further as attaching the handgrip to the distal end of the robotic arm.

In some embodiments, the method further comprises attaching a tool guide to the handgrip, the tool guide having a tool support.

Another aspect of the present disclosure relates to a method comprising: positioning a tool support supported by a robotic arm at a predetermined location with a longitudinal axis of the tool support coaxially aligned with a planned trajectory;
causing a processor of a controller associated with the robotic arm to move the tool support in a first direction along the planned trajectory keeping the longitudinal axis of the tool support substantially coaxially aligned with the at least one planned trajectory;
causing the processor of the controller to move the tool support in a second direction along the at least one planned trajectory keeping the longitudinal axis of the tool support substantially coaxially aligned with the planned trajectory.

In some embodiments, the step of causing the processor of the controller associated with the robotic arm to move the tool guide in the first direction along the planned trajectory keeping the longitudinal axis of the tool support substantially coaxially aligned with the at least one planned trajectory is defined further as receiving, by the processor, a signal from a first trajectory button on the robotic arm thereby providing an instruction to the processor to cause the processor to move the tool support in the first direction along the planned trajectory keeping the longitudinal axis of the tool support substantially coaxially aligned with the at least one planned trajectory.

In some embodiments, the first trajectory button is supported by a body of a handgrip attached to the robotic arm, and the method further comprises receiving a force on the first trajectory button whereby the first trajectory button initiates the signal in response to force on the first trajectory button.

Another object of the present disclosure relates to a method comprising: receiving data indicative of a planned trajectory to be followed by a tool support connected to a robotic arm;
configuring a processor of a controller associated with the robotic arm to move the tool support in a first direction along the planned trajectory keeping the longitudinal axis of the tool support substantially coaxially aligned with the at least one planned trajectory;
configuring the processor of the controller to move the tool support in a second direction along the at least one planned trajectory keeping the longitudinal axis of the tool support substantially coaxially aligned with the planned trajectory.

### BRIEF DESCRIPTION OF THE DRAWINGS

FIG. 1 shows a schematic of a computer-assisted surgical system including a robot base, a robotic arm, a handgrip attached to the robotic arm, and a tool guide attached to the handgrip constructed in accordance with one embodiment of the present disclosure;
FIG. 2 is an illustrative view of a surgical site taken transversely through a patient's vertebra depicting the handgrip and tool guide of FIG. 1 with a tool secured by the tool guide and aligned along a pre-planned trajectory adjacent to the surgical site in accordance with one embodiment of the present disclosure;
FIG. 3 is an exploded front perspective view of the handgrip and tool guide of FIG. 1 constructed in accordance with one embodiment of the present disclosure;
FIG. 4 is an exploded rear perspective view of the handgrip and tool guide of FIG. 1 constructed in accordance with one embodiment of the present disclosure;
FIG. 5 shows a workflow for a surgical procedure which employs a computer-assisted surgical system in accordance with one embodiment of the present disclosure.

### DETAILED DESCRIPTION

Before explaining at least one embodiment of the disclosure in detail, it is to be understood that the disclosure is not limited in its application to the details of construction, experiments, exemplary data, and/or the arrangement of the components set forth in the following description or illustrated in the drawings unless otherwise noted.

The systems and methods as described in the present disclosure are capable of other embodiments or of being practiced or carried out in various ways. Also, it is to be understood that the phraseology and terminology employed herein is for purposes of description, and should not be regarded as limiting.

The following detailed description refers to the accompanying drawings. The same reference numbers in different drawings may identify the same or similar elements.

As used in the description herein, the terms "comprises," "comprising," "includes," "including," "has," "having," or any other variations thereof, are intended to cover a non-exclusive inclusion. For example, unless otherwise noted, a process, method, article, or apparatus that comprises a list of elements is not necessarily limited to only those elements, but may also include other elements not expressly listed or inherent to such process, method, article, or apparatus.

Further, unless expressly stated to the contrary, "or" refers to an inclusive and not to an exclusive "or". For example, a condition A or B is satisfied by one of the following: A is true (or present) and B is false (or not present), A is false (or not present) and B is true (or present), and both A and B are true (or present).

In addition, use of the "a" or "an" are employed to describe elements and components of the embodiments herein. This is done merely for convenience and to give a general sense of the inventive concept. This description should be read to include one or more, and the singular also includes the plural unless it is obvious that it is meant otherwise. Further, use of the term "plurality" is meant to convey "more than one" unless expressly stated to the contrary.

As used herein, the term "substantially" means that the subsequently described event or circumstance completely occurs or that the subsequently described event or circumstance occurs to a great extent or degree. As used herein the qualifier "substantially" is intended to include not only the exact value, amount, degree, orientation, or other qualified characteristic or value, but are intended to include some slight variations due to measuring error, control loop error, manufacturing tolerances, stress exerted on various parts or components, observer error, wear and tear, and combinations thereof. For example, when describing the longitudinal axis of the tool support substantially coaxially aligned with at least one planned trajectory, the term "substantially" refers to alignment within tracking tolerances.

As used herein, any reference to "one embodiment," "an embodiment," "some embodiments," "one example," "for example," or "an example" means that a particular element, feature, structure or characteristic described in connection with the embodiment is included in at least one embodiment. The appearance of the phrase "in some embodiments" or "one example" in various places in the specification is not necessarily all referring to the same embodiment, for example.

Circuitry, as used herein, may be analog and/or digital components, or one or more suitably programmed processors (e.g., microprocessors) and associated hardware and software, or hardwired logic. Also, "components" may perform one or more functions. The term "component" may include hardware, such as a processor (e.g., microprocessor), a combination of hardware and software, and/or the like. Software may include one or more computer executable instructions that when executed by one or more components cause the component to perform a specified function. It should be understood that the algorithms described herein may be stored on one or more non-transitory memory. Exemplary non-transitory memory may include random access memory, read only memory, flash memory, and/or the like. Such non-transitory memory may be electrically based, optically based, and/or the like.

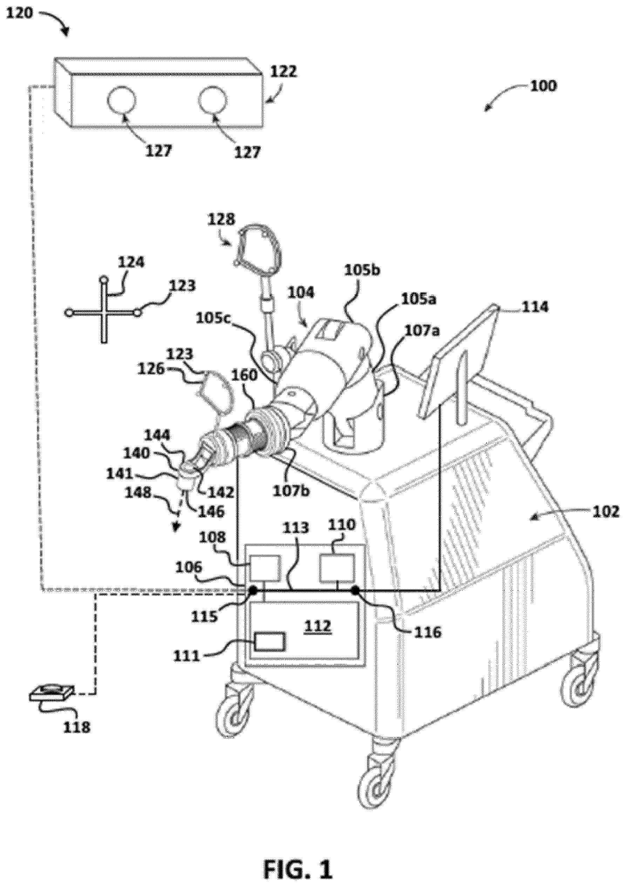

Referring now to the drawings, and in particular to figures 1-4, shown therein is an overview of an exemplary computer-assisted surgical system 100. The computer-assisted surgical system 100 may be provided with a robot base 102 supporting a robotic arm 104. The robot base 102 is depicted as a mobile base, but stationary bases are also contemplated. The robotic arm 104 includes a plurality of arm segments 105a, 105b and 105c connected by rotatable or otherwise articulating joints and may be moved by actuation of the joints. One of the arm segments 105 forms a distal end 107b of the robotic arm 104. In the example shown in FIG. 1, the arm segment 105c of the robotic arm 104 forms the distal end107b. The robotic arm 104 also includes a proximal end 107a attached to and supported by the robot base 102, and the distal end 107b. The robotic arm 104 may be adapted to move in all six degrees of freedom during a surgical procedure. The robotic arm 104 may be configured for incremental changes (e.g., in each of the six degrees of freedom) to ensure the necessary precision during surgery. The robotic arm 104 may actively move about the joints to position the robotic arm 104 in a desired position relative to a patient (not depicted), or the robotic arm 104 may be set and locked into a position. For example, the present disclosure is contemplated to include use of tools by surgical robots, by users with some degree of robotic assistance, and without involvement of surgical robots or robotic assistance (e.g., once positioned and locked).

A control unit or controller 106 enables various features of the system 100, and performance of various methods disclosed herein in accordance with some embodiments of the present disclosure. In some embodiments, the controller 106 can control operation of the robotic arm 104 and associated navigational system(s) 120. In some embodiments, the control may comprise calibration of relative systems of coordinates, generation of planned trajectories, monitoring of position of various units of the robot base 102, and/or units functionally coupled thereto, implementation of safety protocols or limits, and the like. The controller 106 may be a system or systems able to embody and/or execute logic of processes described herein. The controller 106 may be include circuitry configured to execute logic embodied in the form of software instructions and/or firmware. In some embodiments, the logic described herein may be executed in a stand-alone environment such as on the controller 106 and/or logic may be implemented in a networked environment such as a distributed system using multiple computers and/or processors. In some embodiments, the planned trajectory can be based upon a longitudinal axis of an implant to be positioned in a predetermined implant position within the patient. The predetermined implant position may include a position and orientation in 3D space of where the implant will be installed in the patient. In these embodiments, the planned trajectory may coincide with a longitudinal axis of the implant projected from the predetermined implant position within the patient.

The various embodiments of the present disclosure can be operational with other computing systems, environments, and/or configurations that can be suitable for use with the systems and methods of the invention comprise personal computers, server computers, laptop devices or handheld devices, and multiprocessor systems configured to execute logic embodied in the form of software instructions and/or firmware described herein. Additional examples comprise mobile devices, programmable consumer electronics, network PCs, minicomputers, mainframe computers, distributed computing environments that comprise any of the above systems or devices, and the like.

The controller 106 may include one or more processors 108 (hereinafter "processor 108"), one or more communication devices 110 (hereinafter "communication device 110"), one or more non-transitory memory 112 (hereinafter "memory 112") storing processor executable code and/or software application(s), such as application 111, and a system bus 113 that couples various components including the processor 108 to the memory 112, for example.

In general, the processor 108 refers to any computing processing unit or processing device comprising, but not limited to, single-core processors; single-processors with software multithread execution capability; multi-core processors; multi-core processors with software multithread execution capability; multi-core processors with hardware multithread technology; parallel platforms; and parallel platforms with distributed shared memory. Additionally, or alternatively, the processor 108 may be an integrated circuit, an application specific integrated circuit (ASIC), a digital signal processor (DSP), a field programmable gate array (FPGA), a programmable logic controller (PLC), a complex programmable logic device (CPLD), a discrete gate or transistor logic, discrete hardware components, or any combination thereof designed to perform the functions described herein. Processors or processing units referred to herein can exploit nano-scale architectures such as, molecular and quantum-dot based transistors, switches and gates, in order to optimize space usage or enhance performance of the computing devices that can implement the various aspects of the subject invention. In some embodiments, processor 108 also can be implemented as a combination of computing processing units.

An external device 114 may communicate with the controller 106. The external device 114 may be a touch-screen display, a computing device, remote server, etc., configured to allow a surgeon or other user to input data directly into the controller 106. Such data may include patient information and/or surgical procedure information. The external device 114 may display information from the controller 106, such as alerts. Communication between the external device 114 and the controller 106 may be wireless or wired. The illustrated external device 114 is shown attached to the robot base 102, however, in some embodiments, the external device 114 may not be attached to the robot base 102. For example, the external device 114 may be located within a surgical room, but not attached to the robot base 102.

The system 100 may also comprise the navigational system 120 that includes a tracking unit 122. The system 100 is able to monitor, track, and/or determine changes in the relative position and/or orientation of one or more parts of the robotic arm 104, a tool guide 140 attached to the robotic arm 104, and/or a tool inserted in the tool guide 140, as well as various parts of the patient's body B, within a common coordinate system by utilizing various types of fiducials 123 (e.g., multiple degree-of-freedom optical, inertial, and/or ultrasonic sensing devices), navigation systems 120 (e.g., machine vision systems, charge coupled device cameras, tracker sensors, surface scanners, and/or range finders), anatomical computer models (e.g., magnetic resonance imaging scans of the lower lumbar region of the spine), data from previous surgical procedures and/or previously-performed surgical techniques (e.g., data recorded by the system 100 while performing earlier steps of a surgical procedure), and the like. Tracking may be performed in a number of ways, e.g., using stereoscopic optical detectors 127, ultrasonic detectors, sensors configured to receive position information from inertial measurement units, etc. Tracking in real time, in some embodiments, means high frequencies greater than twenty Hertz, in some embodiments in the range of one hundred to five hundred Hertz, with low latency, in some embodiments less than five milliseconds. Regardless of how it is gathered, position and orientation data may be transferred between components (e.g., to the controller 106) via any suitable connection, e.g., with wires or wirelessly using a low latency transfer protocol. The controller 106 may carry out real-time control algorithms at a reasonably high frequency with low additional latency to coordinate movement of the robotic arm 104 of the system 100. The tracking unit 122 may also include cameras, or use the stereoscopic optical detectors 127, to detect, for example, characteristics of the tool guide 140 attached to the robotic arm 104.

Fiducials 123 of the navigational system 120 may be attached to the navigation arrays (e.g., the first navigation array 124, the second navigational array 126, and the optional navigation array 128 (and/or other navigation arrays)). Fiducials 123 may be arranged in predetermined positions and orientations with respect to one another. The fiducials 123 may be aligned to lie in planes of known orientation (e.g., perpendicular planes, etc.) to enable setting of a Cartesian reference frame. The fiducials 123 may be positioned within a field of view of a navigation system 120 and may be identified in images captured by the navigation system 120. The fiducials 123 may be single-use reflective navigation markers. Exemplary fiducials 123 include infrared reflectors, light emitting diodes (LEDs), spherical reflective markers, blinking LEDs, augmented reality markers, and so forth. The first navigation array 124, second navigation array 126, and optional navigation array 128 may be or may include an inertial measurement unit (IMU), an accelerometer, a gyroscope, a magnetometer, other sensors, or combinations thereof. The sensors may transmit position and/or orientation information to the navigation system 120. In other embodiments, the sensors may be configured to transmit position and/or orientation information to an external controller which may be, for example, the controller 106.

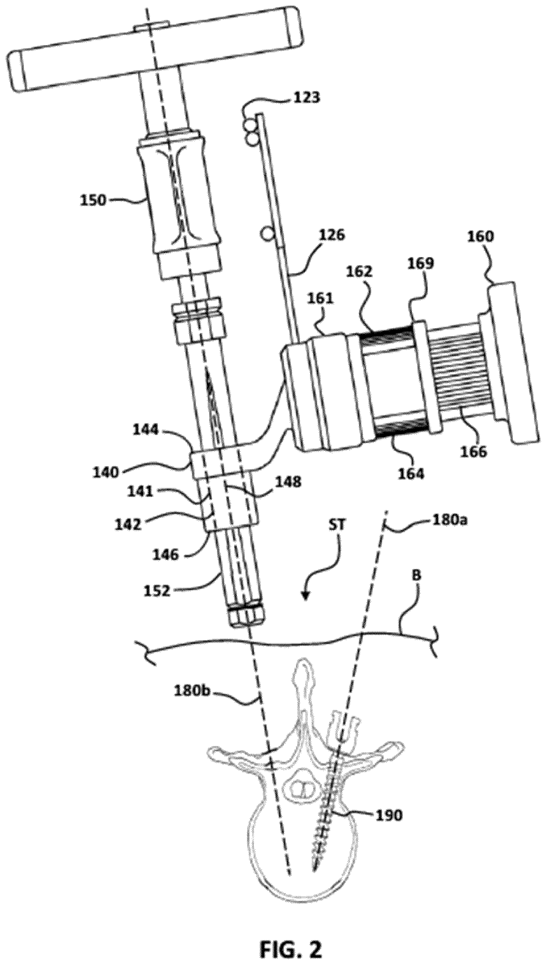

The second navigation array 126 may be mounted on the robotic arm 104, on the handgrip 160, or on the tool guide 140 and may be used to determine a position of the robotic arm 104 or a distal portion thereof (indicative of a position of the tool guide 140). The structure and operation of the second navigation array 126 may vary depending on the type of navigation system 120 used. In some embodiments, the second navigation array 126 may include one or more sphere-shaped or other fiducials 123 for use with an optical navigation system, for example, the second navigation array 126 illustrated in FIG. 2 with the spherical fiducial 123. The navigation system 120 facilitates registering and tracking of the position and/or orientation of the second navigation array 126 and, by extension, the tool guide 140 and a relative distance of the tool guide 140 to other objects in the operating room, e.g., a patient, a surgeon, etc. Position and/or orientation data may be gathered, determined, or otherwise handled by the navigation system 120 using registration/navigation techniques to determine coordinates of each navigation array and/or fiducial 123 within a coordinate system. These coordinates may be communicated to the controller 106 which uses the coordinates of each navigation array and/or fiducial 123 to calculate a position and orientation of the tool guide 140 in the coordinate system and a position of the tool guide 140 relative to the patient to facilitate articulation of the robotic arm 104.

The application 111 may configure the controller 106, or the processor 108 thereof, to perform the automated control of position of the robotic arm 104 in accordance with aspects of the invention. Such control can be enabled, at least in part, by the navigation system 120. In some embodiments, when the controller 106 is functionally coupled to the robotic arm 104, the application 111 can configure the controller 106 to perform the functionality described in the present disclosure. In some embodiments, the application 111 may be retained or stored in memory 112 as a group of computer-accessible instructions (for instance, computer-readable instructions, computer-executable instructions, or computer-readable computer-executable instructions). In some embodiments, the group of computer-accessible instructions can encode the methods of the presently disclosed inventive concepts. In some embodiments, the application 111 may encode various formalisms (e.g., image segmentation) for computer vision tracking using the navigation system 120. In some embodiments, the application 111 may be a compiled instance of such computer-accessible instructions stored in the memory 112, a linked instance of such computer-accessible instructions, a compiled and linked instance of such computer-executable instructions, or an otherwise executable instance of the group of computer-accessible instructions.

The memory 112 may be any available media that is accessible by the controller 106 and comprises, for example and not meant to be limiting, both volatile and non-volatile media, removable and non-removable media. In some embodiments, the memory 112 comprises computer readable media in the form of volatile memory, such as random access memory (RAM), and/or non-volatile memory, such as read only memory (ROM). In some embodiments, the memory 112 may store data (such as a group of tokens employed for code buffers) and/or program modules such as the application 111 that are immediately accessible to, and/or are presently operated-on by the controller 106. In some embodiments, the memory may store an operating system (not shown) such as Windows operating system, Unix, Linux, Symbian, Android, Apple iOS operating system, Chromium, and substantially any operating system for wireless computing devices or tethered computing devices. Apple^{®} is a trademark of Apple Computer, Inc., registered in the United States and other countries. iOS^{®} is a registered trademark of Cisco and used under license by Apple Inc. Microsoft^{®} and Windows^{®} are either registered trademarks or trademarks of Microsoft Corporation in the United States and/or other countries. Android^{®} and Chrome^{®} operating system are registered trademarks of Google Inc. Symbian^{®} is a registered trademark of Symbian Ltd. Linux^{®} is a registered trademark of Linus Torvalds. UNIX^{®} is a registered trademark of The Open Group.

In some embodiments, the memory 112 may be a mass storage device which can provide non-volatile storage of computer code (e.g., computer-executable instructions such as the application 111), computer-readable instructions, data structures, program modules, and other data for the controller 106. For instance, in some embodiments, the memory 112 may be a hard disk, a removable magnetic disk, a removable optical disk, magnetic cassettes or other magnetic storage devices, flash memory cards, CD-ROM, digital versatile disks (DVD) or other optical storage, random access memories (RAM), read only memories (ROM), electrically erasable programmable read-only memory (EEPROM), and the like.

In some embodiments, optionally, any number of program modules can be stored on the memory 112, including by way of example, the operating system, and a tracking software (not shown). In some embodiments, data and code (for example, computer-executable instructions, patient-specific trajectories, and patient anatomical data) may be retained and stored on the memory 112. In some embodiments, data and/or code, may be stored in any of one or more databases known in the art. Examples of such databases comprise, DB2^{®}, Microsoft^{®} Access, Microsoft^{®} SQL Server, Oracle^{®}, mySQL, PostgreSQL, and the like. Further examples include membase databases and flat file databases. The databases can be centralized or distributed across multiple systems.

DB2^{®} is a registered trademark of IBM in the United States.

Microsoft^{®}, Microsoft^{®} Access^{®}, and Microsoft^{®} SQL Server^{™} are either registered trademarks or trademarks of Microsoft Corporation in the United States and/or other countries.

Oracle^{®} is a registered trademark of Oracle Corporation and/or its affiliates.

MySQL^{®} is a registered trademark of MySQL AB in the United States, the European Union and other countries.

PostgreSQL^{®} and the PostgreSQL^{®} logo are trademarks or registered trademarks of The PostgreSQL Global Development Group, in the U.S. and other countries.

In some embodiments, the user (for example, a surgeon or other user, or equipment) can enter commands and information into the controller 106 via the external device 114 using an input device (not shown). Examples of such input devices include, but are not limited to, a keyboard, a pointing device (for example, a mouse), a microphone, a joystick, a scanner (for example, a barcode scanner), a reader device such as a radiofrequency identification (RFID) readers or magnetic stripe readers, gesture-based input devices such as tactile input devices (for example, touch screens, gloves and other body coverings or wearable devices), speech recognition devices, or natural interfaces, and the like.

In some embodiments, the external device 114 may be functionally coupled to the system bus 113 via an interface 116. In some embodiments, the controller 106 may be configured to have more than one external device 114. For example, in some embodiments, the external device 114 may be a monitor, a liquid crystal display, or a projector. Further, in addition to the external device 114, some embodiments may include other output peripheral devices that can comprise components such as speakers (not shown) and a printer (not shown) capable of being connected to the controller 106 via interface 116. In some embodiments, a pointing device, may be either tethered to, or wirelessly coupled to the controller 106 to receive input from the user. In some embodiments, any step and/or result of the methods can be output in any form to an output device such as the external device 114. In some embodiments, the output can be any form of visual representation, including, but not limited to, textual, graphical, animation, audio, tactile, and the like.

In certain embodiments, one or more cameras may be contained or functionally coupled to the navigation system 120, which is functionally coupled to the system bus 113 via an input/output interface 115. Such functional coupling can permit the one or more camera(s) to be coupled to other functional elements of the controller 106. In one embodiment, the input/output interface 115, at least a portion of the system bus 113, and the memory 112 can embody a frame grabber unit that can permit receiving imaging data acquired by at least one of the one or more cameras. In some embodiments, the frame grabber can be an analog frame grabber, a digital frame grabber, or a combination thereof. In some embodiments, where the frame grabber is an analog frame grabber, the processor 108 can provide analog-to-digital conversion functionality and decoder functionality to enable the frame grabber to operate with medical imaging data. Further, in some embodiments, the input/output interface 115 can include circuitry to collect the analog signal received from at least one camera of the one or more cameras. In some embodiments, in response to execution by processor 108, the application 111 may operate the frame grabber to receive imaging data in accordance with various aspects described herein.

The system 100 may be provided with a safety signal generator 118 functionally coupled to the processor 108 via input/output interface 115 and the system bus 113. The safety signal generator 118 may be provided having circuitry configured to monitoring a predetermined location and detect a presence or absence of a part of the surgeon in the predetermined area. For example, in some embodiments, the safety signal generator 118 can include a switch for making and breaking a connection in an electrical circuit which may be positioned within the predetermined area. For example, the switch can be a foot pedal that may be actuated by a foot of the surgeon.

While the system 100 may utilize tool guides of various shapes, sizes, and functionalities, the depicted tool guide 140 has an aperture 142 for retaining, guiding, positioning, supporting, and/or locating at least one tool 150 such as a rotary tool. Advantageously, the tool guide 140 may be configured to guide, position, support, or locate a series of tools 150 used in a surgical procedure, such as spinal surgery, with respect to a surgical site ST. The robotic arm 104 may be configured to help a user (e.g., a surgeon) guide, position, support, or locate the tools 150 along at least one planned trajectory 180 using the tool guide 140. Shown in FIG. 2 by way of example are two planned trajectories 180a and 180b. Exemplary tools 150 include, but are not limited to, a dilator having a dilator tip (e.g., sharp or blunt), a probe, a cutting instrument, a tap, a screw, etc. The cutting instrument may be, for example, a drill, saw blade, burr, reamer, mill, scalpel blade, or any other implement that could cut bone or other tissue and is appropriate for use in a particular surgical procedure. The tools may be secured in the tool guide 140 using a locking mechanism (not shown). The locking mechanism may be a slider locking mechanism or other feature, for instance.

As will be explained below, in some embodiments, signal(s) passed from the first trajectory button 162 to the processor 108 of the controller 106 associated with the robotic arm 104 causes the processor 108 to move the tool support 141 in a first direction (e.g., distal or towards the patient) along the planned trajectory 180 keeping the longitudinal axis 148 of the tool support 141 coaxially aligned with the at least one planned trajectory 180. Signal(s) passed from the second trajectory button 164 to the processor 108 of the controller 106 causes the processor 108 to move the tool support 141 in a second direction (e.g., proximal or away from the patient) along the at least one planned trajectory 180 keeping the longitudinal axis 148 of the tool support 141 coaxially aligned with the planned trajectory 180. In some embodiments, the signals include instructions to cause the processor 108 to move the tool support in the first direction or the second direction.

In some embodiments, the tool guide 140 includes a tool support 141 having an aperture 142 extending from a first face 144 of the tool support 141 to a second face 146 of the tool support 141 and has a longitudinal axis 148 that extends through a center of the aperture 142. In some embodiments, the tool support 141 can be a tube.

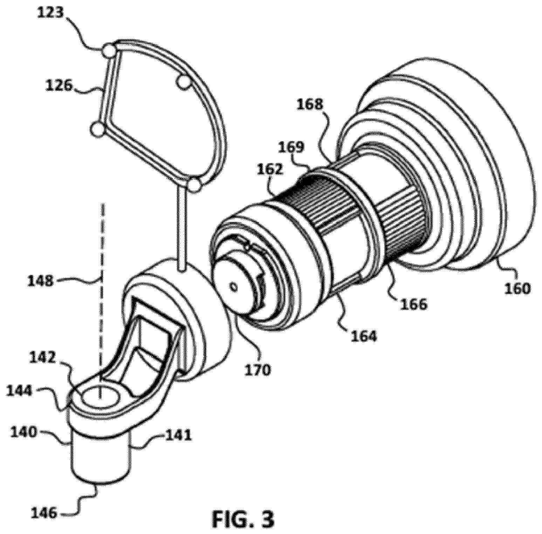

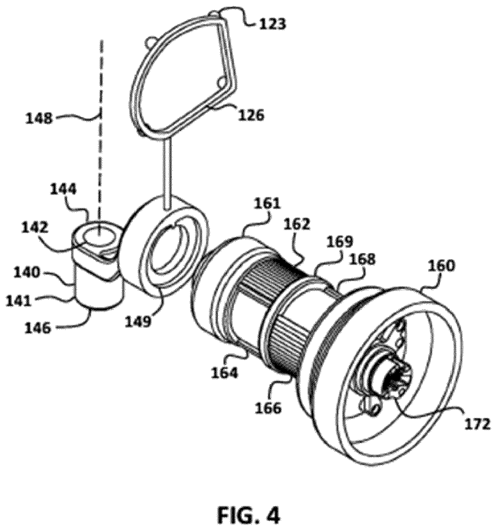

The handgrip 160 may be provided with a body 161 supporting a first trajectory button 162, a second trajectory button 164, a first admittance button 166, and a second admittance button 168. A ridge 169 (see FIG. 2) may be formed in the body 161 separating the first and second trajectory buttons 162 and 164 from the first and second admittance buttons 166 and 168. The handgrip 160 may be provided with a connection portion 170 (see FIG. 3) formed in a distal portion of the body 161 configured to connect the handgrip 160 and the tool guide 140. In the embodiment shown, the connection portion 170 may be a male connector configured to receive the corresponding receiving portion 149 (see FIG. 4) of the tool guide 140. The handgrip 160 may further be provided with a connector 172 (see FIG. 4) configured to electrically connect the handgrip 160 to the robotic arm 104. The connector 172 of the handgrip 160 may be a male connector configured to receive a corresponding female connector on the distal end 107b of the robotic arm 104. One or more threaded connection or a pin can be used to mechanically connect the handgrip 160 to the robotic arm 104.

The first trajectory button 162 and the second trajectory button 164 can be positioned adjacent to each other. Or, as shown in FIG. 3, the first trajectory button 162 and the second trajectory button 64 can be positioned on opposite sides of the body 161. The handgrip 160 may also be provided with the first admittance button 166, and the second admittance button 168 positioned adjacent to each other or opposite to each other on the handgrip 160. The first trajectory button 162, the second trajectory button 164, the first admittance button 166, and the second admittance button 168 can be implemented in a variety of manners. For example. The first trajectory button 162, the second trajectory button 164, the first admittance button 166, and the second admittance button 168 can be implemented as mechanical switch, piezoelectric switch, proximity sensor, haptic control device, a graphical control element on a touchscreen, and combinations thereof, that provides the user with a way to trigger a function of the controller 106. The body may be cylindrically shaped.

The first trajectory button 162, second trajectory button 164, first admittance button 166, and second admittance button 168 may be arranged on the handgrip to provide visual and/or tactile means of identifying the buttons. For instance, the first trajectory button 162 and the second trajectory button 164 may be placed opposite each other on the handgrip 160 with the first admittance button 166 and the second admittance button 168 positioned opposite each other and offset from the first trajectory button 162 and the second trajectory button 164 by ninety degrees. This placement allows the user to identify the buttons by touch. Further, the ridge 169 may be positioned between the sets of buttons to further facilitate tactile identification of the buttons. In some embodiments, the first trajectory button 162 and the second trajectory button 164 may be provided having a first color and the first admittance button 166 and the second admittance button 168 may be provided having a second color different from the first color to visually differentiate the sets of buttons from each other.

The controller 106 and the first trajectory button 162 may be configured to move the robotic arm 104 and by extension the tool guide 140 toward the surgical site ST keeping the longitudinal axis 148 of the tool support 141 of the tool guide 140 aligned with the planned trajectory 180. For instance, the first trajectory button 162 may be electrically connected to the controller 106 such that pressing the first trajectory button 162 passes (e.g., pushes or pulls) an electrical signal to the controller 106 causing the processor 108 of the controller 106 to pass electrical signals to the robotic arm 104 to adjust the position of the robotic arm 104 moving the tool support 141 of the tool guide 140 toward the surgical site ST while keeping the longitudinal axis 148 of the tool support 141 of the tool guide 140 aligned with the planned trajectory 180.

The controller 106 and the second trajectory button 164 may be configured to move the robotic arm 104, and by extension the tool support 141 of the tool guide 140, away from the surgical site ST keeping the longitudinal axis 148 of the tool guide 140 aligned with the planned trajectory 180. For instance, the second trajectory button 164 may be electrically connected to the controller 106 such that pressing the second trajectory button 164 pass an electrical signal to the processor 108 of the controller 106 causing the processor 108 of the controller 106 to pass electrical signals to the robotic arm 104 to adjust the position of the robotic arm 104 moving the tool support 141 of the tool guide 140 away from the surgical site ST while keeping the longitudinal axis 148 of the tool support 141 of the tool guide 140 aligned with the planned trajectory 180.

The controller 106 and the first trajectory button 162 and the second trajectory button 164 may be configured to move the robotic arm 104, and by extension the tool support 141 of the tool guide 140 from a current position (e.g., on planned trajectory 180a) to a next planned trajectory such as planned trajectory 180b. For instance, the first trajectory button 162 and the second trajectory button 164 may be electrically connected to the controller 106 such that pressing or otherwise selecting the first trajectory button 162 and the second trajectory button 164 at substantially the same time pass an electrical signal to the processor 108 of the controller 106 causing the processor 108 to pass electrical signals to the robotic arm 104 to move the robotic arm 104 from a current position (e.g., on planned trajectory 180a) to a position on the next planned trajectory 180b. For instance, during a surgical procedure, once the anchor 190 is placed, the user may press the first trajectory button 162 and the second trajectory button 164 at substantially the same time to move the tool guide 140 from the current position on planned trajectory 180a to a position on planned trajectory 180b with the longitudinal axis 148 of the tool support 141 of the tool guide 140 coaxially aligned with planned trajectory 180a to begin placement of a second anchor.

By way of example, during a surgical procedure, the user may insert the cannula 152 in the tool support 141 of the tool guide 140. Using the information stored about the cannula 152, the processor 108 of the system 100 may move the robotic arm 104 (and hence tool support 141 of the tool guide 140 and cannula 152), such that the cannula 152 arrives at a position a predetermined distance from the body B and/or anatomical structure of the patient. The surgeon may move the tool 150 away from the patient along the trajectory 180 using the second trajectory button 164 to improve visibility or access, for instance. When moving the tool 150 back toward the patient along the trajectory 180 using the first trajectory button 162, the processor 108 of the system 100 will stop the robotic arm 104 when the safety limit has been reached.

In some embodiments, the controller 106 may be operated in a mode, that will be referred to herein as admittance mode, that permits manual positioning of the tool guide 140 by allowing and/or assisting movement of the robotic arm 104 as directed by the user. For instance, the first admittance button 166 and the second admittance button 168 may be configured to pass signals to the processor 108, which processes and pass signals to the robotic arm 104 to sense torque on the robotic arm 104 from the user placing force on the robotic arm 104. The processor 108 determines which direction the user wants to move the robotic arm 104 and then actuates servos within the robotic arm 104 to allow the user to move the robotic arm 104 freely in all directions subject to the safety limits. In admittance mode, the processor 108 of the system 100 may be configured to activate the servos in the robotic arm 104 and sense torque at the servo or servos at each joint in the robotic arm 104 to determine a desired direction the user is attempting to move the robotic arm 104 after which the processor 108 may be programmed to activate one or more servos to assist the user in moving the robotic arm 104 in the desired direction. Admittance mode may be entered when both the first admittance button 166 and the second admittance button 168 are depressed in a predetermined sequence, e.g., at substantially the same time (e.g., simultaneously, within 1 second or the like), and then held simultaneously throughout the movement.

As described herein, some embodiments include the controller 106 that can control operation of the robotic arm 104. The processor 108 of the controller 106 may be configured to execute the application 111 to control the robotic arm 104. In some embodiments, the application 111, in response to execution by the processor 108, can utilize trajectories (such as, tip and tail coordinates) that can be planned and/or configured remotely or locally before and/or during a surgical procedure. A trajectory that has been planned before or during the surgical procedure may be referred to herein as a "-planned trajectory" such as the planned trajectories 180a and 180b. In an additional or alternative aspect, in response to execution by the processor 108, the application 111 may be configured to implement one or more of the methods described herein in the controller 106 to cause movement of the robotic arm 104 according to one or more trajectories. It should be noted that for a spine surgery there are multiple planned trajectories. It would be common to have six trajectories (three pairs of two trajectories, i.e., one pair of trajectories for each vertebral body involved in the surgery). In some embodiments, four trajectories may be used for fusing two vertebral bodies together.

In some embodiments, the processor 108 of the controller 106 may convey the status of the robot arm 104 and/or other device being locked in position using a visual or aural alert. For instance, an LED or other visual device may be included on the handgrip 160 or the tool guide 140 and the controller 106 may be programmed to illuminate the LED to indicate the status of the robot arm 104 and/or other device being locked in position.

In some embodiments, the processor 108 of the controller 106 may be programmed to enable continuous control of tool guide 140 position relative to the anatomy of a patient.

In some embodiments, the processor 108 of the controller 106 may be programmed to enable a user (for example, a surgeon or other user, or equipment) to position conventional surgical screws. In some embodiments, the processor 108 of the controller 106 may be programmed to enable selection of a length and diameter of surgical screws by the user. In yet another aspect, the processor 108 of the controller 106 may be programmed to ensure that a relative position, size, and scale of screws are maintained on the external device 114 when in graphical representation.

The tool guide 140 may be removably coupled to the handgrip 160 which is coupled to the robotic arm 104. As can be appreciated, there should be no play between the tool guide 140 and robotic arm 104. To facilitate coupling the tool guide 140 to the handgrip 160, the tool guide 140 may be provided having the receiving portion 149 sized and shaped to receive the connecting portion 170 of the handgrip 160. The receiving portion 149 and connecting portion 170 are shown for illustration purposes only and should not be considered limiting. The tool guide 140 and the handgrip 160 may be provided having any receiving portion 149 and connecting portion 170 configured to securely couple the tool guide 140 and the handgrip 160. For example, the receiving portion 149 and the connecting portion 170 can included at least one threaded connection, e.g., a screw, configured to securely couple the tool guide 140 and the handgrip 160.

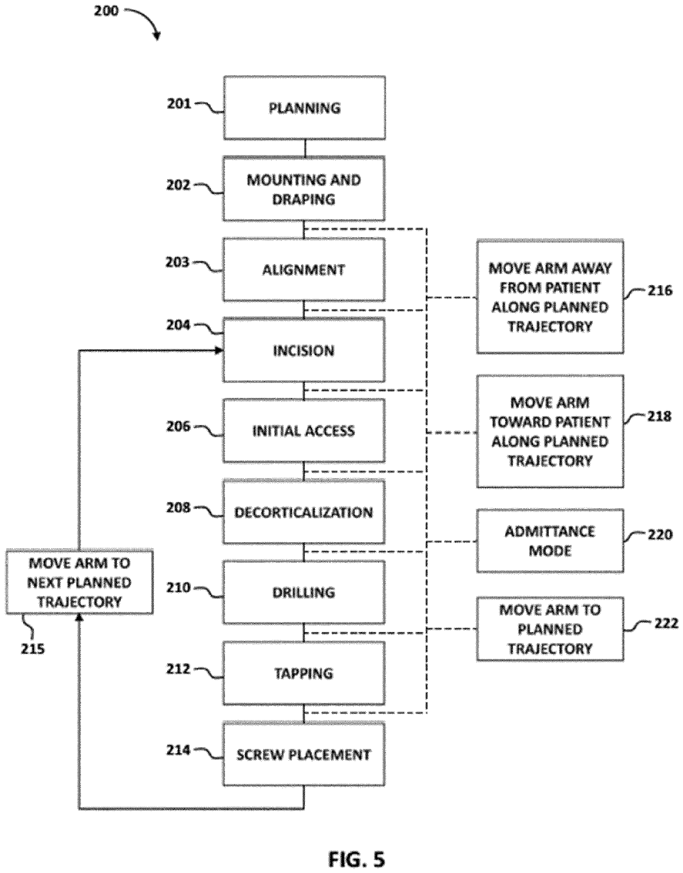

FIG. 5 shows a workflow 200 for a surgical procedure (e.g., pursuant to a treatment plan) which may be employed with a computer-assisted surgical system (e.g., such as the computer-assisted surgical system 100 of FIG. 1. having the robotic arm 104, the controller 106, and the navigational system 120. For example, the surgical procedure may involve a patient's spine, such as placement of screws in one or more pedicles of a patient's vertebrae. By way of a non-limiting example, the surgical procedure may employ a drill, tap, and screw technique, such as may be required as part of a transforaminal lumbar interbody fusion (TLIF) procedure. A series of tools may be required by the surgical procedure. One example of a procedure is a posterior pedicle screw placement for posterior stabilization which is often performed together with an interbody procedure (e.g., placement of a cage).

At step 201, the processor 108 of the controller 106 may be programmed to enable a user to locate an intended position of a surgical implant or tool. For instance, at least one trajectory may be planned to access anatomical structures of the patient. For instance, each trajectory may be planned using imaging of the patient anatomy (e.g., magnetic resonance imaging scans of the lower lumbar region of the spine) that have been used to create anatomical computer models, data from previous surgical procedures and/or previously-performed surgical techniques (e.g., data recorded by the system 100 while forming the pilot holes that are subsequently used to facilitate installation of the anchor 190), and the like. In some embodiments, the user may program a desired point of insertion and trajectory for a surgical instrument to reach a desired anatomical target within or upon the body B of the patient. In some embodiments, the desired point of insertion and trajectory can be planned on the anatomical computer model, which in some embodiments, can be displayed on the external device 114. In some embodiments, the user can plan the trajectory and desired insertion point (if any) on a computed tomography scan (hereinafter referred to as "CT scan") of the patient. In some embodiments, the CT scan can be an isocentric C-arm type scan, an O-arm type scan, or intraoperative CT scan as is known in the art. However, in some embodiments, any known 3D image scan can be used in accordance with the embodiments of the invention described herein. The at least one trajectory planned as described in step 201 may be referred to throughout as a "-planned trajectory" such as the -planned trajectory 180.

In some embodiments, the processor 108 of the controller 106 may be programmed to generate a display that follows a standardized workflow that is planned during step 201. For instance, the surgeon can select in which order the pedicle screws are inserted. Thus, after a first pedicle screw is placed, for instance, when the user presses both the first trajectory button 162 and the second trajectory button 164 at substantially the same time, the processor 108 causes the robotic arm 104 to move from a current position to a next planned trajectory for insertion of a second pedicle screw. The current position of the robotic arm 104 may be on the planned trajectory for placement of the first pedicle screw or the surgeon may have entered admittance mode by pressing both the first admittance button 166 and the second admittance button 168 to move the robotic arm 104

At step 202, the tool guide 140, e.g., having the tool support 141, e.g., a connector or coupler, that is adapted to receive a plurality of tools 150 (e.g., different tools sequentially) is supported (e.g., attached or mounted) to the handgrip 160 that is supported (e.g., mounted or attached) to the distal end 107b or other location on the robotic arm 104 of the computer-assisted surgical system 100. The tool guide 140 may be coupled to the handgrip 160, for example, via an end plate locked by a lever or other coupling means known in the art such as screws, bolts, threadingly connecting, and the like. In other embodiments, the tool guide 140 and the handgrip 160 may be integrally formed as a unitary structure. Prior to attaching the tool guide 140, a sterile drape (not shown) may be placed between the tool guide 140 and the handgrip 160 and extended to cover the handgrip 160 and/or at least part of the robotic arm 104.

In step 203, alignment of the tool guide 104 to the planned trajectory 180 is performed. In one example, after the tool guide 140 is connected to an active robotic arm such as robotic arm 104, navigational assessments using the controller 106 and associated navigational system 120 may be performed to ensure alignment of the tool guide 140. Alignment may be performed with no tool 150 in the tool guide 140, with the tool 150 being a referencing tool in the tool guide 140, or with an initial tool 150 of the surgical procedure in the tool guide 140. In some embodiments, the robotic arm 104 is only aligned once to a planned trajectory (for example, per pedicle screw insertion procedure). With respect to the handgrip 160 and tool guide 140, mounting may only need to be performed once, but with respect to the tool 150 in the surgical procedure (e.g., each tool 150 in the surgical procedure), a mounting and alignment step may be repeated at each tool change and/or distal tip change. A common reason for realignment is a detected deviation from the planned trajectory 180 due to applied forces on the surgical system 100.

At step 204, a first tool 150 (e.g., such as a scalpel) retaining a cutting instrument (e.g., such as a scalpel blade) is placed in the tool guide 140. A scalpel guide may be placed in the tool support 141 of the tool guide 140 to guide a scalpel holder (e.g., with blade) and therefore allow an opening of the patient in the pre-planned trajectory given by the aligned (based on pre-planning) tool guide 140. Optionally, step 203 may be performed with respect to alignment of the first tool 150 (e.g., at a desired trajectory, position, and/or orientation). The robotic arm 104 may navigate to a starting position (a system with an active robot arm) or may be guided to the starting position (a system with a passive arm with an active tool guide) by a user (e.g., a surgeon). The navigational system 120 and/or the processor 108 of the controller 106 may store the position (e.g., a three-dimensional position). A cutting trajectory may be displayed on the external device 114, along with imaging of the patient anatomy, etc. An incision is made in the patient to access the surgical site ST. The incision may be made manually by the surgeon. The incision may be made semi-autonomously, that is, controlled with at least partial robotic control (e.g., force assist from a robot, robotic constraint against movement beyond certain bounds or in certain planes while otherwise providing for manual control, determining to keep on a straight line, depth control, etc.). The incision may be made fully autonomously (e.g., controlled entirely by the controller 106). Once the incision at the surgical site ST is complete, patient anatomical structures such as a bone surface, are accessible. The navigational system 120 and/or the processor 108 of the controller 106 may store a position (e.g., a three-dimensional position) of the robotic arm 104 and/or tool support 141 of the tool guide 140. For example, this position may include an incision boundary, or an incision depth determination. The first tool 150 may be removed from the tool support 141 of the tool guide 140.

At step 206, a second tool 150 (e.g., such as a dilator) retaining a cutting instrument (e.g., such as a sharp dilator tip (or, alternatively, a blunt dilator tip)) is placed in the tool support 141 of the tool guide 140 and secured in place. This allows for initial access to the anatomical structures at the surgical site ST. Optionally, step 203 may be performed with respect to alignment of the second tool 150 (e.g., at a desired trajectory, position, and/or orientation). For example, the robotic arm 104 (and hence tool support 141 of the tool guide 140), may be returned to a stored position. Imaging of the patient anatomy may be displayed on the external device 114. The second tool 150 is inserted into the patient to access the surgical site ST. Control of the second tool 150 may be made manually, semi-autonomously, or fully autonomously, as described previously. In some embodiments, the initial access is a dilation procedure. Once the dilation procedure is complete, the navigational system 120 and/or the processor 108 of the controller 106 may store a position (e.g., a three-dimensional position) of the robotic arm 104 and/or the tool support 141 of the tool guide 140. For example, this position may include a depth determination that indicates a position of a bone surface. The second tool 150 may be removed from the tool support 141 of the tool guide 140.

At step 208, a third tool 150 (e.g., such as for driving a burr) retaining a cutting instrument (e.g., such as a burr) is placed in the tool support 141 of the tool guide 140 and secured in place. The burr may be one of a variety of shapes, e.g., flat, round, or geometrical, with geometrical or non-geometrical cutting structures. Moreover, the burr may have a variety of configurations, e.g., fluted or non-fluted. Fluted burrs may have different numbers of cutting flutes. Optionally, step 203 may be performed with respect to alignment of the third tool 150 (e.g., at a desired trajectory, position, and/or orientation). For example, the robotic arm 104 (and hence tool support 141 of the tool guide 140), may be moved such that the third tool 150 arrives at a stored position (e.g., a position on a bone surface). Imaging of the patient anatomy may be displayed on the external device 114. The burr is inserted into the patient to access the surgical site ST and create a flat or other feature on the bone surface. The robotic arm 104 may move along the planned trajectory. Control of a rotary tool driving the burr may be made manually, semi-autonomously, or fully autonomously. Once the decorticalization procedure is complete, the navigational system 120 and/or the processor 108 of the controller 106 may store a position (e.g., a three-dimensional position) of the robotic arm 104 and/or the tool support 141 of the tool guide 140 in the memory 112. For example, this position may include a depth determination that indicates a position and/or contour of the decorticalized bone surface. The third tool 150 may be removed from the tool support 141 of the tool guide 140. As may be appreciated, in some embodiments, this step may be omitted depending on a type of screw to be installed.

At step 210, a fourth tool 150 (e.g., such as for driving a drill bit) retaining a cutting instrument (e.g., such as a drill bit) is placed in the tool support 141 of the tool guide 140 and retained in place. The drill bit may be one of a variety of diameters, lengths, and/or configurations, e.g., fluted or non-fluted. Optionally, step 203 may be performed with respect to alignment of the fourth tool 150 (e.g., at a desired trajectory, position, and/or orientation). For example, the robotic arm 104 (and hence tool guide 140), may be moved such that the fourth tool 150 arrives at a stored position (e.g., a position on a bone surface). Imaging of the patient anatomy may be displayed on the external device 114. The drill bit is inserted into the patient to access the surgical site ST and create a bore in the bone. The robotic arm 104 may move to achieve the planned trajectory 180. Control of the rotary tool driving the drill bit may be made manually, semi-autonomously, or fully autonomously, as described previously. Once the drilling procedure is complete, the navigational system 120 and/or the processor 108 of the controller 106 may store a position (e.g., a three-dimensional position) of the robotic arm 104 and/or the tool support 141 of the tool guide 140 in the memory 112. For example, this position may include a depth determination that indicates a position of the bore. The fourth tool 150 may be removed from the tool support 141 of the tool guide 140. As may be appreciated, in some embodiments, this step may be omitted depending on a type of screw to be installed.

At step 212, a fifth tool 150 (e.g., such as for driving a tap) retaining a cutting instrument (e.g., such as a tap) is placed in the tool support 141 of the tool guide 140 and secured in place. The tap may be selected based on a predetermined screw size (e.g., to be inserted into the bore at step 214). Optionally, step 203 may be performed with respect to alignment of the fifth tool (e.g., at a desired trajectory, position, and/or orientation). For example, the robotic arm 104 (and hence tool guide 140), may be moved such that the fifth tool 150 arrives at a stored position (e.g., a position on a bone surface). Imaging of the patient anatomy may be displayed on the external device 114. The tap is inserted into the patient to access the surgical site ST and create threads in the bore in the bone. The robotic arm 104 may move to and/or along the pre-planned trajectory. Control of the rotary tool driving the tap may be made manually, semi-autonomously, or fully autonomously, as described previously. Once the tapping procedure is complete, the navigational system 120 and/or the processor 108 of the controller 106 may store a position (e.g., a three-dimensional position) of the robotic arm 104 and/or the tool support 141 of the tool guide 140 in the memory 112. For example, this position may include a depth determination that indicates a position of the tapped bore. The fifth tool 150 may be removed from the tool support 141 of the tool guide 140. As may be appreciated, in some embodiments, this step may be omitted depending on a type of screw to be installed.

At step 214, a sixth tool 150 (e.g., such as for driving a screw) retaining a cutting instrument (e.g., such as a screw) is placed in the tool support 141 of the tool guide 140 and secured in place. The screw may be a pedicle screw of a predetermined screw size (e.g., to be inserted into the tapped bore). Optionally, step 203 may be performed with respect to alignment of the sixth tool (e.g., at a desired trajectory, position, and/or orientation). For example, the robotic arm 104 (and hence tool support 141 of the tool guide 140), may be moved such that the sixth tool 150 arrives at a stored position (e.g., a position on a bone surface). Imaging of the patient anatomy may be displayed on the external device 114. The screw is inserted into the patient to access the surgical site ST and be installed in the bore in the bone. The robotic arm 104 may move to and/or along the pre-planned trajectory. Control of the sixth tool 150 driving the screw may be made manually, semi-autonomously, or fully autonomously, as described previously. Once the screw installation procedure is complete, the navigational system 120 and/or the processor 108 of the controller 106 may store a position (e.g., a three-dimensional position) of the robotic arm 104 and/or the tool support 141 of the tool guide 140. For example, this position may include a placement determination that indicates a position of the pedicle screw. The sixth tool 150 may be removed from the tool support 141 of the tool guide 140.

When the surgical procedure includes multiple planned trajectories, such as a surgical procedure requiring placement of multiple pedicle screws, the pedicle screw in step 214 may be a first pedicle screw that is placed along a first planned trajectory. After the first pedicle screw is placed, at step 215 the user may move the robotic arm 104 from a current position, which may be on the first planned trajectory, to a second planned trajectory for placing a second pedicle screw, by pressing the first trajectory button 162 and the second trajectory button 164 at substantially the same time. The workflow 200 may be repeated for the second pedicle screw beginning at step 204, for instance.

When the tool holder 141 is aligned with the planned trajectory 180, at optional step 216, the user may move the robotic arm 104 and the tool guide 140 holding the tool 150 toward the patient by pushing or otherwise selecting the first trajectory button 162 on the handgrip 160. During this movement, the processor 108 of the system 100 is programmed to move the robotic arm 104 such that the longitudinal axis 148 of the tool support 141 of the tool guide 140, and by association the tool, stays aligned with the pre-planned trajectory. Once the tool holder is aligned with the planned trajectory 180, optional step 216 may be performed at any time during the surgical procedure, for instance, to improve visibility and/or access to the surgical site ST. In some embodiments, the first trajectory button 162 must be pressed or otherwise selected during the entire movement of the robotic arm 104.

In some embodiments, the system 100 may be programmed to require that the first trajectory button 162 and the safety signal generator 118 be pushed or otherwise selected in a predetermined sequence, e.g., at the same time (simultaneously), in a predetermined order, or combinations thereof before the robotic arm 104 and the tool guide 140 holding the tool 150 are moved toward the patient. In some embodiments, the system 100 may be programmed to require the safety signal generator 118 to be pushed or otherwise selected before the first trajectory button 162, and then both of the safety signal generator 118 and the first trajectory button 162 may be pushed or otherwise selected simultaneously before the robotic arm 104 and the tool guide 140 holding the tool 150 are moved toward the patient.

When the tool holder 141 is aligned with the planned trajectory 180, at optional step 218, the user may move the robotic arm 104 and the tool support 141 of the tool guide 140 holding the tool 150 away from the patient by pushing or otherwise selecting the second trajectory button 164 on the handgrip 160. During this movement, the system 100 is programmed to move the robotic arm 104 such that the longitudinal axis 148 of the tool support 141 of the tool guide 140, and by association the tool 150, stays aligned with the planned trajectory 180. Optional step 216 may be performed at any time during the surgical procedure, for instance, to improve visibility and/or access to the surgical site ST. In some embodiments, the second trajectory button 164 must be pressed or otherwise selected during the entire movement of the robotic arm 104.

In some embodiments, the system 100 may be programmed to require that the second trajectory button 164 and the safety signal generator 118 be pushed or otherwise selected at the same time before the robotic arm 104 and the tool guide 140 holding the tool 150 are moved away from the patient.

At optional step 220, the user may move the robotic arm 104 and the tool support 141 of the tool guide 140 holding the tool 150 in various directions (including but not limited to away from the planned trajectory 180), subject to the safety limits discussed above, by pressing or otherwise selecting the first admittance button 166 and the second admittance button 168 at substantially the same time and holding the first admittance button 166 and the second admittance button 168 during the movement of the robotic arm 104. Optional step 220 may be performed at any time during the surgical procedure, for instance, to improve visibility and/or access to the surgical site ST. Upon releasing the first admittance button 166 and/or the second admittance button 168, the processor 108 may be configured to move the robotic arm 104 to place the longitudinal axis 148 of the tool guide 140, and by association the tool 150 with the planned trajectory 180.

At optional step 222, the user may move the robotic arm 104 and the tool support 141 of the tool guide 140 holding the tool 150 from a current position away from the planned trajectory to the planned trajectory 180 by pressing or otherwise selecting the first trajectory button 162 and the second trajectory button 164 at substantially the same time. Optional step 222 may be performed at any time during the surgical procedure when the tool support 141 is not aligned with the planned trajectory. For instance, this can occur when the surgeon may enter admittance mode as discussed above in step 220 by pressing both the first admittance button 166 and the second admittance button 168 to move the robotic arm 104 around obstacles after which the user may press the first trajectory button 162 and the second trajectory button 164 at substantially the same time to return the tool support 141 of the tool guide 140 to the planned trajectory 180 while other parts of the robotic arm 104 do not block the surgeon's view.

As can be appreciated, the presently described embodiments may also be applicable to other surgical procedures such as cervical procedures, etc.

From the above description, it is clear that the inventive concept(s) disclosed herein are well adapted to carry out the objects and to attain the advantages mentioned herein, as well as those inherent in the inventive concept(s) disclosed herein. While the embodiments of the inventive concept(s) disclosed herein have been described for purposes of this disclosure, it will be understood that numerous changes may be made and readily suggested to those skilled in the art which are accomplished within the scope and spirit of the inventive concept(s) disclosed herein.

## Claims

1. A robotic surgical system including a robotic arm (104), the robotic surgical system comprising:
a handgrip (160) supported by the robotic arm (104), the handgrip having a first trajectory button (162) and a second trajectory button (164);
a tool guide (140) supported by the handgrip (160), the tool guide comprising a tool support (141) having a first end, a second end, an aperture extending through the tool support (141) from the first end to the second end, and a longitudinal axis (148) extending through a center of the aperture from the first end to the second end; and
a controller (106) in communication with the robotic arm (104) and the handgrip (160), the controller having a non-transitory computer readable memory and a processor, the non-transitory computer readable memory storing at least one planned trajectory associated with a surgical procedure and processor executable instructions that, when executed, cause the processor to pass a first signal to the robotic arm causing the robotic arm (104) to position the tool support (141) at a predetermined distance from a patient with the longitudinal axis (148) of the tool support substantially coaxially aligned with the at least one planned trajectory;
wherein the first trajectory button (162), when engaged, is configured to pass a second signal to the processor of the controller causing the processor of the controller to move the tool support away from the patient along the at least one planned trajectory keeping the longitudinal axis (148) of the tool support substantially coaxially aligned with the at least one planned trajectory and the second trajectory button (164), when engaged, is configured to pass a third signal to the processor of the controller causing the processor of the controller to move the tool support toward the patient along the at least one planned trajectory keeping the longitudinal axis (148) of the tool support substantially coaxially aligned with the at least one planned trajectory.

2. The robotic surgical system of claim 1, further comprising a tool (150) positioned in the tool support (141).

3. The robotic surgical system of claim 2, wherein the non-transitory computer readable memory of the controller (106) further stores a safety limit associated with the surgical procedure, the safety limit configured to restrict movement of the tool (150) secured in the tool support (141) such that a predetermined distance limit between the tool and anatomy of a patient is maintained during the surgical procedure.

4. The robotic surgical system of any one of claims 1 to 3, wherein the handgrip (160) further comprises a first admittance button (166) and a second admittance button (168), the first admittance button and second admittance button, when engaged at substantially the same time, are configured to pass a fourth signal to the controller (106) causing the controller to allow a user to manually move the robotic arm (104) in various directions.

5. The robotic surgical system of any one of claims 1 to 4, further comprising a navigation system (120) having a tracking unit (122) and a navigation array (126) attached to the robotic arm (104), the navigation system in communication with the controller (106), wherein the navigation system is configured to track a position and orientation of the tool guide (140) using the navigation array and pass the position and orientation of the tool guide to the controller.

6. The robotic surgical system of any one of claims 1 to 5, further comprising a sterile drape disposed between the tool guide (140) and the handgrip (160) and extending to cover the handgrip during a surgical procedure.

7. The robotic surgical system of any one of claims 1 to 6, wherein the first trajectory button (162) and the second trajectory button (164), when engaged in a predetermined sequence, are configured to pass a fourth signal to the processor of the controller (106) causing the processor of the controller to move the tool guide (140) from a current position to a new position with the longitudinal axis of the tool guide coaxially aligned with a second planned trajectory.

8. The robotic surgical system of any one of claims 1 to 7, wherein the first trajectory button (162) and the second trajectory button (164) are positioned opposite each other on the handgrip (160).

9. The robotic surgical system of claim 8, wherein the first admittance button (166) and second admittance button (168) are positioned opposite each other on the handgrip (160) with the first admittance button offset from the first trajectory button.

10. The robotic surgical system of claim 4, wherein the handgrip (160) further comprises a body (161) supporting the first trajectory button (162), the second trajectory button (164), the first admittance button (166) and the second admittance button (168), and wherein the body of the handgrip comprises a ridge (169) positioned to separate the first trajectory button and the second trajectory button from the first admittance button and second admittance button.

11. The robotic surgical system of claim 4, wherein the first trajectory button (162) and the second trajectory button (164) are provided having a first color and the first admittance button (166) and second admittance button (168) are provided having a second color different from the first color.

12. The robotic surgical system of any one of claims 1 to 11, further comprising a safety signal generator having circuitry configured to monitor a predetermined location and detect a presence or absence of a part of the surgeon in the predetermined area, and wherein the processor is programmed to require the safety signal generator to be pushed or otherwise selected in a predetermined sequence with the second trajectory button (164) to cause the processor of the controller (106) to move the tool support toward the patient along the at least one planned trajectory.

13. The robotic surgical system of any one of claims 1 to 11, further comprising a safety signal generator having circuitry configured to monitor a predetermined location and detect a presence or absence of a part of the surgeon in the predetermined area, and wherein the processor is programmed to require the safety signal generator to be pushed or otherwise selected in a predetermined sequence with the first trajectory button (162) to cause the processor of the controller (106) to move the tool support away from the patient along the at least one planned trajectory.

14. A method of assembling a surgical robotic system according to any one of claims 1 to 13, comprising:
attaching a handgrip (160) to at least one arm segment of a robotic arm (104), the handgrip having a body (161) supporting a first trajectory button (162) and a second trajectory button (164).

15. The method of claim 14, wherein one (105c) of the arm segments of the robotic arm (104) forms a distal end (107b) of the robotic arm, and wherein the step of attaching is defined further as attaching the handgrip (160) to the distal end (107b) of the robotic arm (104).

16. The method of claim 14 or claim 15, further comprising attaching a tool guide (140) to the handgrip (160), the tool guide having a tool support.
